# EUROPEAN PATENT SPECIFICATION

(11) **EP 1 594 969 B1**
(45) Date of publication and mention of the grant of the patent: **20.05.2015**
(21) Application number: 04707166.7
(22) Date of filing: 31.01.2004
(51) Int. Cl.: C07K 16/18, A61K 39/00

(54) **ACTIVE IMMUNIZATION TO GENERATE ANTIBODIES TO SOLUBLE A-BETA**
AKTIVE IMMUNISIERUNG ZUR ERZEUGUNG VON ANTIKÖRPERN GEGEN LÖSLICHES A-BETA
IMMUNISATION ACTIVE PERMETTANT DE PRODUIRE DES ANTICORPS CONTRE A-BETA SOLUBLE

(30) Priority: 01.02.2003 US 444150 P
(43) Date of publication of application: 16.11.2005
(73) Proprietor: Janssen Sciences Ireland UC, Little Island, County Cork (IE); Wyeth LLC, New York, NY 10017 (US)
(72) Inventor: YEDNOCK, Ted, Forest Knolls, CA 94933 (US); VASQUEZ, Nicki, San Francisco, CA 94114 (US); SEUBERT, Peter, A., San Francisco, CA 94114 (US); BARD, Frederique, Pacifica, CA 94044 (US)
(74) Representative: Kirsch, Susan Edith
(86) International application number: PCT/US2004/002856
(87) International publication number: WO 2004/069182

(56) References cited:
- WO-A-02/096937
- US-A1- 2002 094 335
- US-A1- 2002 162 129
- PALLITTO MONICA M ET AL: "Recognition sequence design for peptidyl modulators of beta-amyloid aggregation and toxicity" BIOCHEMISTRY, AMERICAN CHEMICAL SOCIETY. EASTON, PA, US, vol. 38, no. 12, 23 March 1999 (1999-03-23), pages 3570-3578, XP002152181 ISSN: 0006-2960
- DEMATOS R B ET AL: "Peripheral anti-Abeta antibody alters CNS and plasma Abeta clearance and decreases brain Abeta burden in a mouse model of Alzheimer's disease" PROCEEDINGS OF THE NATIONAL ACADEMY OF SCIENCES OF USA, NATIONAL ACADEMY OF SCIENCE, WASHINGTON, DC, US, vol. 98, no. 15, 17 July 2001 (2001-07-17), pages 8850-8855, XP002969676 ISSN: 0027-8424
- SCHENK D ET AL: "Current progress in beta-amyloid immunotherapy" CURRENT OPINION IN IMMUNOLOGY, ELSEVIER, OXFORD, GB, vol. 16, no. 5, October 2004 (2004-10), pages 599-606, XP004549620 ISSN: 0952-7915
- HARDY J: "AMYLOID, THE PRESENILINS AND ALZHEIMER'S DISEASE" TRENDS IN NEUROSCIENCE, ELSEVIER, AMSTERDAM, NL, 1997, pages 154-159, XP002922840 ISSN: 0166-2236
- SOLOMON B.: 'Immunological approaches as therapy for Alzheimer's disease' VACCINES & ANTIBODIES vol. 2, no. 8, December 2002, pages 907 - 917, XP002971402
- BARD F. ET AL.: 'Epitope and isotype specificities of antobodies to beta-amyloid peptide for protection against Alzheimer's disease-like neuropathology' PNAS vol. 100, no. 4, 18 February 2003, pages 2023 - 2028, XP002982464
- LEVERONE J.F. ET AL.: 'A beta 1-15 is less immunogenic than A beta 1-40/42 for intranasal immunization of wild-type mice but may be effective for "boosting"' VACCINE vol. 21, no. 17-18, 16 May 2003, pages 2206 - 2215, XP004421138
- HOLTZMAN D.M. ET AL.: 'A beta immunization and anti-A beta antibodies: potential therapies for the prevention and treatment of Alzheimer's disease' ADVANCED DRUG DELIVERY REVIEWS vol. 54, no. 12, 07 December 2002, pages 1603 - 1613, XP002982465
- SIGURDSSON E.M.: 'A safer vaccine for Alzheimer's disease?' NEUROBIOLOGY OF AGING vol. 23, no. 6, November 2002 - December 2002, pages 1001 - 1008, XP002982466
- SCHENK D. ET AL.: 'Immunization with amyloid-beta attenuates Alzheimer-disease-like pathology in the PDAPP mouse' NATURE vol. 400, no. 6740, 08 July 1999, pages 173 - 177, XP002154168
- SIGURDSSON E.M. ET AL.: 'Immunization with a nontoxic/nonfibrillar amyloid-beta homologous peptide reduces Alzheimer's disease-associated pathology in transgenic mice' AMERICAN JOURNAL OF PATHOLOGY vol. 159, no. 2, August 2001, pages 439 - 447, XP008000840
- LEMERE C.A. ET AL.: 'Intranasal immunotherapy for the treatment of Alzheimer's disease: Escherichia coli LT and LT(R192G) as muconasal adjuvants' NEUROBIOLOGY OF AGING vol. 23, no. 6, November 2002 - December 2002, pages 991 - 1000, XP002982467

## Description

### TECHNICAL FIELD

The invention resides in the technical fields of immunology and medicine.

### BACKGROUND OF THE INVENTION

Alzheimer's disease (AD) is a progressive disease resulting in senile dementia. *See generally* Selkoe, TINS 16, 403-409 (1993); Hardy et al., WO 92/13069; Selkoe, J. Neuropathol. Exp. Neurol. 53, 438-447 (1994); Duff et al., Nature 373, 476-477 (1995); Games et al., Nature 373, 523 (1995). Broadly speaking, the disease falls into two categories: late onset, which occurs in old age (65 + years) and early onset, which develops well before the senile period, *i.e.,* between 35 and 60 years. In both types of disease, the pathology is the same but the abnormalities tend to be more severe and widespread in cases beginning at an earlier age. The disease is characterized by at least two types of lesions in the brain, senile plaques and neurofibrillary tangles. Senile plaques are areas of disorganized neuropil up to 150 µm across with extracellular amyloid deposits at the center visible by microscopic analysis of sections of brain tissue. Neurofibrillary tangles are intracellular deposits of microtubule associated tau protein consisting of two filaments twisted about each other in pairs.

The principal constituent of the plaques is a peptide termed Aβ or β-amyloid peptide. Aβ peptide is an internal fragment of 39-43 amino acids of a precursor protein termed amyloid precursor protein (APP). Several mutations within the APP protein have been correlated with the presence of Alzheimer's disease. See, *e.g.,* Goate et al., Nature 349, 704) (1991) (valine⁷¹⁷ to isoleucine); Chartier Harlan et al. Nature 353, 844 (1991)) (valine⁷¹⁷ to glycine); Murrell et al., Science 254, 97 (1991) (valine⁷¹⁷ to phenylalanine); Mullan et al., Nature Genet. 1, 345 (1992) (a double mutation changing lysine⁵⁹⁵-methionine⁵⁹⁶ to asparagine⁵⁹⁵-leucine⁵⁹⁶). Such mutations are thought to cause Alzheimer's disease by increased or altered processing of APP to Aβ, particularly processing of APP to increased amounts of the long form of Aβ (*i.e.,* Aβ1-42 and Aβ1-43). Mutations in other genes, such as the presenilin genes, PS1 and PS2, are thought indirectly to affect processing of APP to generate increased amounts of long form Aβ (see Hardy, TINS 20, 154 (1997)). These observations indicate that Aβ, and particularly its long form, is a causative element in Alzheimer's disease.

Immunization of transgenic mouse models of AD with β-amyloid peptide (Aβ) derived-immunogens results in an antibody response that inhibits formation and/or clear amyloid plaques in brains of the mice (Schenk et al., (1999) Nature 400, 173-177; Janus et al., (2000) Nature 408, 979-982, Morgan et al. (2000) Nature 408, 982-985, Sigurdsson et al., (2001) Am. J. Pathol. 159, 439-447.1-4)). Passively administered antibodies to Aβ have achieve similar effects. Antibody-mediated, Fc-dependent phagocytosis by microglial cells and/or macrophages has been proposed as one mechanism for clearance of existing amyloid plaques (Bard et al., (2000) Nat. Med., 6, 916-919 )). This proposal is based on the result that certain peripherally administered antibodies against Aβ enter the CNS of transgenic mice, decorate amyloid plaques, and induce plaque clearance. Also, a strong correlation has been reported between antibodies that were efficacious *in vivo* and in an *ex vivo* assay using sections of PDAPP or Alzheimer's disease (AD) brain to measure plaque clearing activity. Fc receptors on microglial cells effected the clearance response in the ex vivo assay. However, it has been also been reported that antibody efficacy can also be obtained *in vivo* by mechanisms that are independent of Fc interactions (Bacskai et al., (2002) J. Neurosci., 22, 7873-7878). An antibody directed against the mid-portion of Aβ, which cannot recognize amyloid plaques, was reported to bind to soluble Aβ and reduce plaque deposition (DeMattos et al., (2001) Proc. Natl. Acad. Sci. USA, 98, 8850-8855). Short-term treatment with this antibody has also been reported to improve performance in an object recognition task without affecting amyloid burden (Dodart et al., (2002) Nat. Neurosci., 5, 452-457). Pallitto et al. (Biochemistry. 1999 Mar 23;38(12):3570-8) describes a modular strategy for generating compounds that inhibit Aβ toxicity, based on linking a recognition element for Aβ to a disrupting element designed to interfere with Aβ aggregation. WO-A-02/096937 describes a stereochemically based 'non-self' antigen vaccine for the prevention and/or treatment of Alzheimer's and other amyloid related diseases. US 2002/0162129 A1 describes the use of a non-wild type protofibril or compound(s) with protofibril forming activity for active immunization in the purpose of treating or preventing AD. US 2002/0094335 A1 discloses a stereochemically based "non-self" antigen vaccine for the prevention and/or treatment of Alzheimer's and other amyloid related diseases. Lemere, C.A. et al., (Neurobiology of aging. 2002 Nov/Dec 23(6) 991-1000) describes an Abeta vaccine protocol in AD mouse models with the expectation that higher Abeta antibody titers may be more effective in reducing cerebral Abeta levels.

This application is related to WO 00/72880 filed May 26, 2000, WO 99/27944, filed November 30, 1998, U.S. Application No. 60/067,740, filed December 2, 1997, U.S. Application No. 60/080,970, filed April 7, 1998, and U.S. Application No. 09/201,430, filed November 30, 1998.

### BRIEF DESCRIPTION OF THE DRAWINGS

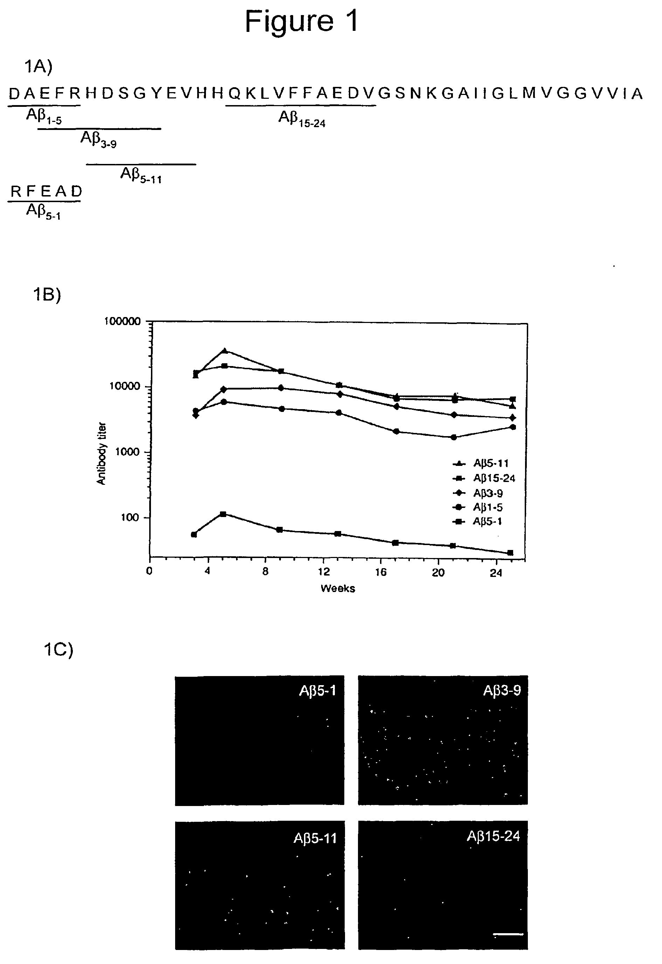

Figs. 1A-C. Antibodies produced by immunization with N-terminal fragments of Aβ bind to amyloid plaques. Fig. 1A. Peptides encompassing various domains of Aβ1-42 (SEQ ID NO:1) (synthesized contiguous to T cell epitope derived from ovalbumin) were used to immunize PDAPP mice. A reversemer, Aβ5-1 (SEQ ID NO:2), was used as a negative control. Fig. 1B. ELISA titers against aggregated Aβ1-42 were significantly higher over the length of the study in the Aβ5-11 and Aβ15-24 groups than in the Aβ1-5 group (1:14,457, p<0.01 and 1:12,257, p<0.05 vs. 1:3,647, respectively; ANOVA followed by *post hoc* Tukey's test). Fig. 1C. Unfixed cryostat sections from untreated PDAPP mouse brain were exposed to the sera of mice immunized with Aβ5-1, Aβ3-9, Aβ5-11, or Aβ15-24 (titers normalized to 1:1000 for staining). Antibodies to Aβ15-24 did not bind to amyloid plaques. Scale bar represents 500µm.

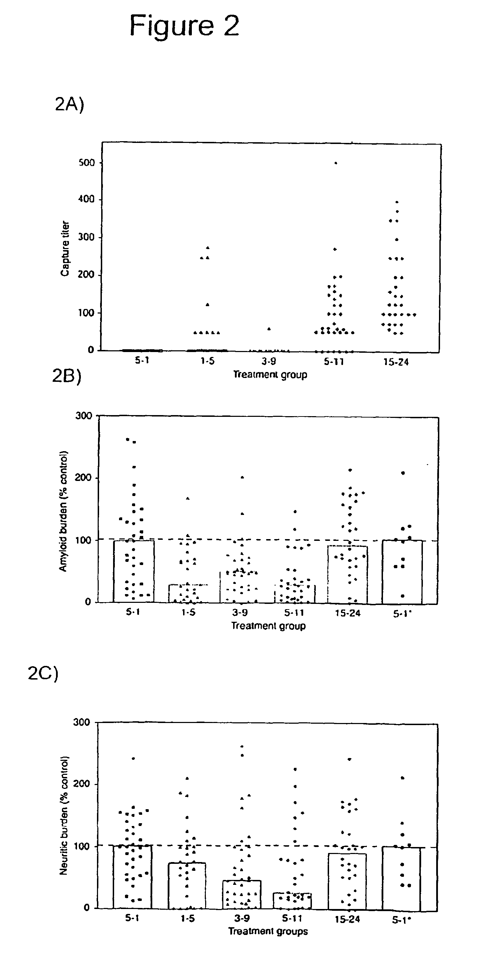

Figs. 2A-C. Capture of soluble Aβ1-42 by antibodies is not associated with reduced amyloid burden or neuritic pathology. Fig. 2A. Sera from mice immunized with fragments of Aβ were examined for their ability to capture radiolabeled soluble Aβ1-42 in a radioimmunoassay. Sera from all animals immunized with Aβ15-24 were able to capture soluble Aβ1-42 (one serum sample had a titer higher than 1:1,350 and a precise titer was not determined), compared with 27% of those in the Aβ1-5 group and 3% of the Aβ3-9 group. Figs.2B-C. Amyloid burden (Fig. 2B) and neuritic pathology (Fig. 2C) were evaluated with image analysis by a blinded microscopist. Values are expressed as a percentage of the mean of the Aβ5-1 group (negative control reversemer peptide). The Aβ5-11 group was evaluated at a separate sitting from the other groups, but in conjunction with the same negative control group as an internal reference (second Aβ5-1 reversemer set, on the left). Amyloid burden was significantly reduced in the Aβ1-5, Aβ3-9, and Aβ5-11 groups (p<0.001. Bars represent median values and the dashed horizontal line indicates the control level. Neuritic burden was significantly reduced in the Aβ3-9 and Aβ5-11 groups (p<0.05). Neither endpoint was significantly altered by immunization with Aβ15-24 group. Statistical analysis was preformed with square root transformation (to normalize non-parametric distributions), and analyzed with ANOVA. A Dunnett's test was then used to compare the multiple groups Aβ1-5, Aβ3-9, Aβ15-24 groups with their Aβ5-1 control, and Mann-Whitney for the Aβ5-11 group with its corresponding Aβ5-1 reversemer control.

### DETAILED DESCRIPTION

### I. General

The invention provides a medicament comprising a fragment of Aβ consisting of Aβ16-23 (KLVFFAED) in the natural human amino acid form, wherein the fragment is linked to a carrier molecule to form a conjugate which helps elicit an immune response against the fragment, for effecting prophylaxis of or treating a disease associated with amyloid deposits of Aβ in the brain of a patient, whereby the induced antibodies specifically bind to soluble Aβ in the patient thereby inhibiting formation of amyloid deposits of Aβ in the brain from the soluble Aβ and thereby effecting treatment or prophylaxis of the disease.

### II. DEFINITIONS

For purposes of classifying amino acids substitutions as conservative or nonconservative, amino acids are grouped as follows: Group I (hydrophobic sidechains): norleucine, met, ala, val, leu, ile; Group II (neutral hydrophilic side chains): cys, ser, thr; Group III (acidic side chains): asp, glu; Group IV (basic side chains): asn, gln, his, lys, arg; Group V (residues influencing chain orientation): gly, pro; and Group VI (aromatic side chains): trp, tyr, phe. Conservative substitutions involve substitutions between amino acids in the same class. Non-conservative substitutions constitute exchanging a member of one of these classes for a member of another.

The term "all-D" refers to peptides having ≥ 75%, ≥ 80%, ≥ 85%, ≥ 90%, ≥ 95%, and 100% D-configuration amino acids.

The term "agent" is used to describe a compound that has or may have a pharmacological activity. Agents include compounds that are known drugs, compounds for which pharmacological activity has been identified but which are undergoing further therapeutic evaluation, and compounds that are members of collections and libraries that are to be screened for a pharmacological activity.

Therapeutic agents herein disclosed are typically substantially pure from undesired contaminant. This means that an agent is typically at least about 50% w/w (weight/weight) purity, as well as being substantially free from interfering proteins and contaminants. Sometimes the agents are at least about 80% w/w and, more preferably at least 90 or about 95% w/w purity. However, using conventional protein purification techniques, homogeneous peptides of at least 99% w/w can be obtained. Therapeutic agents herein disclosed may prevent, effect prophylaxis of, or treat a disease associated with amyloid deposits.

Specific binding between two entities means the entities have a mutual affinity for each other that is at least 10-, 100- or 100-fold greater than the affinity of either entity for a control, such as unrelated antigen or antibody to a different antigen. The mutual affinity of the two entities for each other is usually at least 10⁷, 10⁸, 10⁹ M⁻¹, or 10¹⁰ M⁻¹. Affinities greater than 10⁸ M⁻¹ are preferred. Specific binding of a polyclonal antibody to an epitope within Aβ means the antibodies in the polyclonal antibody population specifically bind to one epitope of Aβ without binding to other epitopes of Aβ.

The term "antibody" or "immunoglobulin" is used to include intact antibodies and binding fragments thereof. Typically, fragments compete with the intact antibody from which they were derived for specific binding to an antigen fragment including separate heavy chains, light chains Fab, Fab' F(ab')2, Fabc, and Fv. Fragments are produced by recombinant DNA techniques, or by enzymatic or chemical separation of intact immunoglobulins. The term "antibody" also includes one or more immunoglobulin chains that are chemically conjugated to, or expressed as, fusion proteins with other proteins. The term "antibody" also includes bispecific antibody. A bispecific or bifunctional antibody is an artificial hybrid antibody having two different heavy/light chain pairs and two different binding sites. Bispecific antibodies can be produced by a variety of methods including fusion of hybridomas or linking of Fab' fragments. See, *e.g.,* Songsivilai & Lachmann, Clin. Exp. Immunol. 79:315-321 (1990); Kostelny et al., J. Immunol. 148, 1547-1553 (1992).

Aβ, also known as β-amyloid peptide, or A4 peptide (see US 4,666,829; Glenner & Wong, Biochem. Biophys. Res. Commun., 120, 1131 (1984)), is a peptide of 39-43 amino acids, which is the principal component of characteristic plaques of Alzheimer's disease. Aβ has several natural occurring forms. The natural human forms of Aβ are referred to as Aβ39, Aβ40, Aβ41, Aβ42 and Aβ43. The sequences of these peptides and their relationship to the APP precursor are illustrated by Fig. 1 of Hardy et al., TINS 20, 155-158 (1997). For example, Aβ42 has the sequence:

H2N--
-
-OH (SEQ ID NO:1).

Aβ41, Aβ40 and Aβ39 differ from Aβ42 by the omission of Ala, Ala-Ile, and Ala-Ile-Val respectively from the C-terminal end. Aβ43 differs from Aβ42 by the presence of a threonine residue at the C-terminus.

APP⁶⁹⁵, APP⁷⁵¹, and APP⁷⁷⁰ refer, respectively, to the 695, 751, and 770 amino acid residue long polypeptides encoded by the human APP gene. See Kang et al., Nature, 325, 773 (1987); Ponte et al., Nature, 331, 525 (1988); and Kitaguchi et al., Nature, 331, 530 (1988). Amino acids within the human amyloid precursor protein (APP) are assigned numbers according to the sequence of the APP770 isoform. Terms such as Aβ39, Aβ40, Aβ41, Aβ42 and Aβ43 refer to an Aβ peptide containing amino acid residues 1-39, 1-40, 1-41, 1-42 and 1-43, respectively.

Disaggregated Aβ or fragments thereof means monomeric peptide units. Disaggregated Aβ or fragments thereof are generally soluble, and are capable of self-aggregating to form soluble oligomers. Oligomers of Aβ and fragments thereof are usually soluble and exist predominantly as alpha-helices or random coils. One method to prepare monomeric Aβ is to dissolve lyophilized peptide in neat DMSO with sonication. The resulting solution is centrifuged to remove any insoluble particulates. Aggregated Aβ or fragments thereof, means oligomers of Aβ or immunogenic fragments thereof in which the monomeric units are held together by noncovalent bonds and associate into insoluble beta-sheet assemblies. Aggregated Aβ or fragments thereof, means also means fibrillar polymers. Fibrils are usually insoluble. Some antibodies bind either soluble Aβ or fragments thereof or aggregated Aβ or fragments thereof. Some antibodies bind both soluble Aβ or fragments thereof and aggregated Aβ or fragments thereof. Some antibodies bind to soluble Aβ without binding to plaque.

An "antigen" is an entity to which an antibody specifically binds.

The term "epitope" or "antigenic determinant" refers to a site on an antigen to which B and/or T cells respond. B-cell epitopes can be formed both from contiguous amino acids or noncontiguous amino acids juxtaposed by tertiary folding of a protein. Epitopes formed from contiguous amino acids are typically retained on exposure to denaturing solvents whereas epitopes formed by tertiary folding are typically lost on treatment with denaturing solvents. An epitope typically includes at least 3, and more usually, at least 5 or 8-10 amino acids in a unique spatial conformation. Methods of determining spatial conformation of epitopes include, for example, x-ray crystallography and 2-dimensional nuclear magnetic resonance. See, *e.g.,* Epitope Mapping Protocols in Methods in Molecular Biology, Vol. 66, Glenn E. Morris, Ed. (1996). Antibodies that recognize the same epitope can be identified in a simple immunoassay showing the ability of one antibody to block the binding of another antibody to a target antigen. T-cells recognize continuous epitopes of about nine amino acids for CD8 cells or about 13-15 amino acids for CD4 cells. T cells that recognize the epitope can be identified by *in vitro* assays that measure antigen-dependent proliferation, as determined by ³H-thymidine incorporation by primed T cells in response to an epitope (Burke et al., J. Inf. Dis., 170, 1110-19 (1994)), by antigen-dependent killing (cytotoxic T lymphocyte assay, Tigges et al., J. Immunol., 156, 3901-3910) or by cytokine secretion.

An N-terminal epitope of Aβ means an epitope with residues 1-11. An epitope within a C-terminal region means an epitope within residues 29-43, and an epitope within a central regions means an epitope with residues 12-28.

The term "immunological" or "immune" response is the development of a beneficial humoral (antibody mediated) and/or a cellular (mediated by antigen-specific T cells or their secretion products) response directed against an amyloid peptide in a recipient patient. Such a response can be an active response induced by administration of immunogen or a passive response induced by administration of antibody or primed T-cells. A cellular immune response is elicited by the presentation of polypeptide epitopes in association with Class I or Class II MHC molecules to activate antigen-specific CD4⁺ T helper cells and/or CD8⁺ cytotoxic T cells. The response may also involve activation of monocytes, macrophages, NK cells, basophils, dendritic cells, astrocytes, microglia cells, eosinophils or other components of innate immunity. The presence of a cell-mediated immunological response can be determined by proliferation assays (CD4⁺ T cells) or CTL (cytotoxic T lymphocyte) assays (see Burke, *supra*; Tigges, *supra*). The relative contributions of humoral and cellular responses to the protective or therapeutic effect of an immunogen can be distinguished by separately isolating antibodies and T-cells from an immunized syngeneic animal and measuring protective or therapeutic effect in a second subject.

An "immunogenic agent" or "immunogen" is capable of inducing an immunological response against itself on administration to a mammal, optionally in conjunction with an adjuvant.

The term "naked polynucleotide" refers to a polynucleotide not complexed with colloidal materials. Naked polynucleotides are sometimes cloned in a plasmid vector.

The term "adjuvant" refers to a compound that when administered in conjunction with an antigen augments the immune response to the antigen, but when administered alone does not generate an immune response to the antigen. Adjuvants can augment an immune response by several mechanisms including lymphocyte recruitment, stimulation of B and/or T cells, and stimulation of macrophages.

The term "patient" includes human and other mammalian subjects that receive either prophylactic or therapeutic treatment.

Competition between antibodies is determined by an assay in which the immunoglobulin under test inhibits specific binding of a reference antibody to a common antigen, such as Aβ. Numerous types of competitive binding assays are known, for example: solid phase direct or indirect radioimmunoassay (RIA), solid phase direct or indirect enzyme immunoassay (EIA), sandwich competition assay (see Stahli et al., Methods in Enzymology, 9:242-253 (1983)); solid phase direct biotin-avidin EIA (see Kirkland et al., J. Immunol. 137:3614-3619 (1986)); solid phase direct labeled assay, solid phase direct labeled sandwich assay (see Harlow and Lane, "Antibodies, A Laboratory Manual," Cold Spring Harbor Press (1988)); solid phase direct label RIA using I-125 label (see Morel et al., Molec. Immunol. 25(1):7-15 (1988)); solid phase direct biotin-avidin EIA (Cheung et al., Virology, 176:546-552 (1990)); and direct labeled RIA (Moldenhauer et al., Scand. J. Immunol., 32:77-82 (1990)). Typically, such an assay involves the use of purified antigen bound to a solid surface or cells bearing either of these, an unlabelled test immunoglobulin and a labeled reference immunoglobulin. Competitive inhibition is measured by determining the amount of label bound to the solid surface or cells in the presence of the test immunoglobulin. Usually the test immunoglobulin is present in excess. Antibodies identified by competition assay (competing antibodies) include antibodies binding to the same epitope as the reference antibody and antibodies binding to an adjacent epitope sufficiently proximal to the epitope bound by the reference antibody for steric hindrance to occur. Usually, when a competing antibody is present in excess, it will inhibit specific binding of a reference antibody to a common antigen by at least 50 or 75%.

An antibody that specifically binds to soluble Aβ means an antibody that binds to soluble Aβ with an affinity of at least 10⁷ M⁻¹. Some antibodies bind to soluble Aβ with affinities between 10⁸ M⁻¹ and 10¹¹ M⁻¹.

An antibody that specifically binds to soluble Aβ without specifically binding to plaques means an antibody that binds to soluble Aβ as described above and has at least a ten fold and usually at least 100-fold lower specific binding affinity for plaques *(i.e.,* Aβ in aggregated β-pleated sheet form) from a cadaver of a former Alzheimer's patient or a transgenic animal model. For example, such an antibody might bind to soluble Aβ with an affinity of 10⁹ M⁻¹ and to plaques with an affinity less than 10⁷ M⁻¹. The affinity of such antibodies for plaques is usually less than 10⁷ or 10⁶ M⁻¹. Such antibodies are additionally or alternatively defined by fluorescence intensity relative to an irrelevant control antibody (e.g., an antibody or mixture of polyclonal antibodies to a reversemer Aβ peptide) when the antibodies are contacted with plaques and binding assessed by fluorescently labeling (as described in the Examples section). The fluorescence intensity of antibodies that bind to soluble Aβ peptide without binding to plaques is within a factor of five, sometimes within a factor of two and sometimes indistinguishable within experimental error from that of the control antibody.

Compositions or methods "comprising" one or more recited elements may include other elements not specifically recited. For example, a composition that comprises Aβ peptide encompasses both an isolated Aβ peptide and Aβ peptide as a component of a larger polypeptide sequence.

### III. Aβ Peptides for Active Immunization

Aβ peptides for use in the methods of the disclosure are immunogenic peptides that on administration to a human patient or animal generate antibodies that specifically bind to one or more epitopes between residues 12 and 43 of Aβ without generating antibodies that specifically bind to one or more epitopes within residues 1-11 of Aβ. Antibodies specifically binding to epitopes between residues 12 and 43 specifically bind to soluble Aβ without binding to plaques of Aβ. These types of antibody can specifically bind to soluble Aβ in the circulation of a patient or model amyloid without specifically binding to plaques of Aβ deposits in the brain of the patient or model. The specifically binding of antibodies to soluble Aβ inhibits the Aβ from being incorporated into plaques thus either inhibiting development of the plaques in a patient or inhibiting a further increase in the size or frequency of plaques if such plaques have already developed before treatment is administered.

Preferably, the fragment of Aβ administered lacks an epitope that would generate a T-cell response to the fragment. Generally, T-cell epitopes are greater than 10 contiguous amino acids. Therefore, preferred fragments of Aβ are of size 5-10 or preferably 7-10 contiguous amino acids; *i.e.,* sufficient length to generate an antibody response without generating a T-cell response. Absence of T-cell epitopes is preferred because these epitopes are not needed for immunogenic activity of fragments, and may cause an undesired inflammatory response in a subset of patients (Anderson et al., (2002) J. Immunol. 168, 3697-3701; Senior (2002) Lancet Neurol. 1, 3). In some of the disclosed methods, the fragment is a fragment other than Aβ13-28, 17-28, 25-35, 35-40, 33-42 or 35-42. Most T-cell epitopes occur within amino acids 14-30 of Aβ.

Fragment Aβ15-24 and subfragments of 7-9 contiguous amino acids thereof are preferred because these peptides consistently generate a high immunogenic response to Aβ peptide. These fragments include Aβ15-21, Aβ16-22, Aβ17-23, Aβ18-24, Aβ19-25, Aβ15-22, Aβ16-23, Aβ17-24, Aβ18-25, Aβ115-23, Aβ16-24, Aβ17-25, Aβ18-26, Aβ15-24, Aβ16-25, and Aβ15-25. The invention relates to Aβ16-23. The designation Aβ15-21 for example, indicates a fragment including residues 15-21 of Aβ and lacking other residues of Aβ. Also disclosed are C-terminal fragments of Aβ42 or 43 of 5-10 and preferably 7-10 contiguous amino acids. These fragments can generate an antibody response that includes end-specific antibodies. These antibodies are advantageous in specifically binding to Aβ42 and Aβ43 without specifically binding to Aβ39-41. These antibodies bind to soluble Aβ without binding to plaque.

In some methods, a fragment from the central or C-terminal region of Aβ is administered in a regime that also includes administering a fragment from the N-terminal region. In general, such fragments induce antibodies that specifically bind to and induce clearing of amyloid plaques via phagocytic cells. Such a response is particularly useful to clear existing deposits of Aβ. However, once the deposits have been cleared, further treatment with a fragment from the central or C-terminal region of Aβ to induce antibodies to soluble Aβ is advantageous for preventing further deposition of Aβ without risk of inflammatory side effects in certain patients. N-terminal fragments beginning at residues 1-3 of Aβ and ending at residues 7-11 of Aβ are particularly preferred. Exemplary N-terminal fragments include Aβ1-5, 1-6, 1-7, 1-10, 3-7, 1-3, and 1-4.

Unless otherwise indicated, reference to Aβ includes the natural human amino acid sequences indicated above as well as analogs including allelic, species and induced variants. Analogs of Aβ induce antibodies that specifically bind with a natural Aβ peptide (e.g., Aβ42). Analogs of Aβ typically differ from naturally occurring peptides at up to 30% of amino acid positions by up to 1, 2, 3, 4, 5, 6, 7, 8, 9 or 10 position changes. Each deletion or substitution of a natural amino acid residue is considered a position change as is the insertion of a residue without substitution. Amino acids substitutions are often conservative substitutions.

Unless otherwise indicated, reference to Aβ fragments includes fragments of the natural human amino acid sequences indicated above as well as analogs including allelic, species and induced variants. Analogs of Aβ fragments induce antibodies that specifically bind with a natural Aβ peptide (e.g., Aβ42). Analogs of Aβ fragments typically differ from naturally occurring peptide fragment at up to about 30% of amino acid positions. For example, an analog of Aβ15-21 may vary by up to 1, 2, 3 or 4 10 position changes. Each deletion or substitution of a natural amino acid residue is considered a position change as is the insertion of a residue without substitution. Amino acids substitutions are often conservative substitutions.

Some analogs of Aβ or Aβ fragments also include unnatural amino acids or modifications ofN or C terminal amino acids at a one, two, five, ten or even all positions. For example, the natural aspartic acid residue at position 1 and/or 7 of Aβ can be replaced with iso-aspartic acid. Examples of unnatural amino acids are D, alpha, alpha-disubstituted amino acids, N-alkyl amino acids, lactic acid, 4-hydroxyproline, gamma-carboxyglutamate, epsilon-N,N,N-trimethyllysine, epsilon-N-acetyllysine, O-phosphoserine, N-acetylserine, N-formylmethionine, 3-methylhistidine, 5-hydroxylysine, omega-N-methylarginine, β-alanine, ornithine, norleucine, norvaline, hydroxproline, thyroxine, gamma-amino butyric acid, homoserine, citrulline, and isoaspartic acid. Some therapeutic agents herein disclosed are all-D peptides, *e.g*., all-D Aβ or all-D Aβ fragment, and all-D peptide analogs. Fragments and analogs can be screened for prophylactic or therapeutic efficacy in transgenic animal models in comparison with untreated or placebo controls as described below.

Aβ, its fragments, and analogs can be synthesized by solid phase peptide synthesis or recombinant expression, or can be obtained from natural sources. Automatic peptide synthesizers are commercially available from numerous suppliers, such as Applied Biosystems, Foster City, California. Recombinant expression can be in bacteria, such as E. coli, yeast, insect cells or mammalian cells. Procedures for recombinant expression are described by Sambrook et al., Molecular Cloning: A Laboratory Manual (C.S.H.P. Press, NY 2d ed., 1989). Some forms of Aβ peptide are also available commercially (e.g., American Peptides Company, Inc., Sunnyvale, CA and California Peptide Research, Inc. Napa, CA).

Therapeutic agents also include longer polypeptides that include, for example, an immunogenic fragment of Aβ peptide, together with one or more other amino acids flanking the Aβ peptide one or one or both sides. For example, preferred agents include fusion proteins comprising a segment of Aβ fused to a heterologous amino acid sequence that induces a helper T-cell response against the heterologous amino acid sequence and thereby a B-cell response against the Aβ segment. One or more flanking heterologous amino acids can also be used to cap an Aβ peptide to protect it from degradation in manufacture, storage or use. Such polypeptides can be screened for prophylactic or therapeutic efficacy in animal models in comparison with untreated or placebo controls as described below. Therapeutic agents herein disclosed include an immunogenic fragment of Aβ flanked by polylysine sequences. The polylysine sequences can be fused to the N-terminus, the C terminus, or both the N- and C-terminus of Aβ or an immunogenic fragment of Aβ. The Aβ peptide, analog, active fragment or other polypeptide can be administered in associated or multimeric form or in dissociated form Therapeutic agents also include multimers of monomeric immunogenic agents.

In a further variation, an immunogenic fragment of Aβ can be presented by a virus or a bacteria as part of an immunogenic composition. A nucleic acid encoding the immunogenic peptide is incorporated into a genome or episome of the virus or bacteria. Optionally, the nucleic acid is incorporated in such a manner that the immunogenic peptide is expressed as a secreted protein or as a fusion protein with an outer surface protein of a virus or a transmembrane protein of a bacteria so that the peptide is displayed. Viruses or bacteria used in such methods should be nonpathogenic or attenuated. Suitable viruses include adenovirus, HSV, Venezuelan equine encephalitis virus and other alpha viruses, vesicular stomatitis virus, and other rhabdo viruses, vaccinia and fowl pox. Suitable bacteria include Salmonella and Shigella. Fusion of an immunogenic peptide to HBsAg of HBV is particularly suitable.

Therapeutic agents of the disclosure also include peptides and other compounds that do not necessarily have a significant amino acid sequence similarity with Aβ but nevertheless serve as mimetics of Aβ and induce a similar immune response. For example, any peptides and proteins forming β-pleated sheets can be screened for suitability. Anti-idiotypic antibodies against monoclonal antibodies to Aβ or other amyloidogenic peptides can also be used. Such anti-Id antibodies mimic the antigen and generate an immune response to it (see Essential Immunology (Roit ed., Blackwell Scientific Publications, Palo Alto, 6th ed.), p. 181). Agents other than Aβ peptides should induce an immunogenic response against one or more of the preferred segments of Aβ listed above (e.g., 15-24). Preferably, such agents induce an immunogenic response that is specifically directed to one of these segments without being directed to other segments of Aβ.

Random libraries of peptides or other compounds can also be screened for suitability. Combinatorial libraries can be produced for many types of compounds that can be synthesized in a step-by-step fashion. Such compounds include polypeptides, beta-turn mimetics, polysaccharides, phospholipids, hormones, prostaglandins, steroids, aromatic compounds, heterocyclic compounds, benzodiazepines, oligomeric N-substituted glycines and oligocarbamates. Large combinatorial libraries of the compounds can be constructed by the encoded synthetic libraries (ESL) method described in Affymax, WO 95/12608, Affymax, WO 93/06121, Columbia University, WO 94/08051, Pharmacopeia, WO 95/35503 and Scripps, WO 95/30642. Peptide libraries can also be generated by phage display methods. See, *e.g*., Devlin, WO 91/18980.

Combinatorial libraries and other compounds are initially screened for suitability by determining their capacity to specifically bind to antibodies or lymphocytes (B or T) known to be specific for Aβ or other amyloidogenic peptides. For example, initial screens can be performed with any polyclonal sera or monoclonal antibody to Aβ or a fragment thereof. Compounds can then be screened for specifically binding to a specific epitope within Aβ *(e.g.,* 15-24). Compounds can be tested by the same procedures described for mapping antibody epitope specificities. Compounds identified by such screens are then further analyzed for capacity to induce antibodies or reactive lymphocytes to Aβ or fragments thereof. For example, multiple dilutions of sera can be tested on microtiter plates that have been precoated with Aβ or a fragment thereof and a standard ELISA can be performed to test for reactive antibodies to Aβ or the fragment. Compounds can then be tested for prophylactic and therapeutic efficacy in transgenic animals predisposed to an amyloidogenic disease, as described in the Examples. Such animals include, for example, mice bearing a 717 mutation of APP described by Games et al., supra, and mice bearing a 670/671 Swedish mutation of APP such as described by McConlogue et al., US 5,612,486 and Hsiao et al., Science, 274, 99 (1996); Staufenbiel et al., Proc. Natl. Acad. Sci. USA, 94:13287-13292 (1997); Sturchler-Pierrat et al., Proc. Natl. Acad. Sci. USA, 94:13287-13292 (1997); Borchelt et al., Neuron, 19:939-945 (1997)). The same screening approach can be used on other potential agents analogs of Aβ and longer peptides including fragments of Aβ, described above.

### IV. Conjugates

Some agents for inducing an immune response contain the appropriate epitope for inducing an immune response against LBs but are too small to be immunogenic. In this situation, a peptide immunogen can be linked to a suitable carrier molecule to form a conjugate which helps elicit an immune response. A single agent can be linked to a single carrier, multiple copies of an agent can be linked to multiple copies of a carrier, which are in turn linked to each other, multiple copies of an agent can be linked to a single copy of a carrier, or a single copy of an agent can be linked to multiple copies of a carrier, or different carriers. Suitable carriers include serum albumins, keyhole limpet hemocyanin, immunoglobulin molecules, thyroglobulin, ovalbumin, tetanus toxoid, or a toxoid from other pathogenic bacteria, such as diphtheria, *E. coli,* cholera, or *H. pylori,* or an attenuated toxin derivative. T cell epitopes are also suitable carrier molecules. Some conjugates can be formed by linking agents herein disclosed to an immunostimulatory polymer molecule (e.g., tripalmitoyl-S-glycerine cysteine (Pam₃Cys), mannan (a manose polymer), or glucan (a beta 1→2 polymer)), cytokines (e.g., IL-1, IL-1 alpha and beta peptides, IL-2, gamma-INF, IL-10, GM-CSF), and chemokines (e.g., MIP1alpha and beta, and RANTES). Immunogenic agents can also be linked to peptides that enhance transport across tissues, as described in O'Mahony, WO 97/17613 and WO 97/17614. Immunogens may be linked to the carries with or with out spacers amino acids (*e.g.,* gly-gly).

Some conjugates can be formed by linking agents herein disclosed to at least one T cell epitope. Some T cell epitopes are promiscuous while other T cell epitopes are universal. Promiscuous T cell epitopes are capable of enhancing the induction of T cell immunity in a wide variety of subjects displaying various HLA types. In contrast to promiscuous T cell epitopes, universal T cell epitopes are capable of enhancing the induction of T cell immunity in a large percentage, *e.g*., at least 75%, of subjects displaying various HLA molecules encoded by different HLA-DR alleles.

A large number of naturally occurring T-cell epitopes exist, such as, tetanus toxoid *(e.g.,* the P2 and P30 epitopes), Hepatitis B surface antigen, pertussis, toxoid, measles virus F protein, *Chlamydia trachomitis* major outer membrane protein, diphtheria toxoid, *Plasmodium falciparum* circumsporozite T, *Plasmodium falciparum* CS antigen, *Schistosoma mansoni* triose phosphate isomersae, *Escherichia coli* TraT, and Influenza virus hemagluttinin (HA). The immunogenic peptides herein disclosed can also be conjugated to the T-cell epitopes described in Sinigaglia F. et al., Nature, 336:778-780 (1988); Chicz R.M. et al., J. Exp. Med., 178:27-47 (1993); Hammer J. et al., Cell 74:197-203 (1993); Falk K. et al., Immunogenetics, 39:230-242 (1994); WO 98/23635; and, Southwood S. et al. J. Immunology, 160:3363-3373 (1998). Further examples include:
Influenza Hemagluttinin: HA₃₀₇₋₃₁₉
Malaria CS: T3 epitope EKKIAKMEKASSVFNV (SEQ ID NO:4)
Hepatitis B surface antigen: HBsAg₁₉₋₂₈ FFLLTRILTI (SEQ ID NO:5)
Heat Shock Protein 65: hsp65₁₅₃₋₁₇₁ DQSIGDLIAEAMDKVGNEG (SEQ ID NO:6)
bacille Calmette-Guerin QVHFQPLPPAVVKL (SEQ ID NO:7)
Tetanus toxoid: TT₈₃₀₋₈₄₄ QYIKANSKFIGITEL (SEQ ID NO:8)
Tetanus toxoid: TT₉₄₇₋₉₆₇ FNNFTVSFWLRVPKVSASHLE (SEQ ID NO:9)
HIV gp120 T1: KQIINMWQEVGKAMYA (SEQ ID NO:10).

Alternatively, the conjugates can be formed by linking agents herein disclosed to at least one artificial T-cell epitope capable of binding a large proportion of MHC Class II molecules., such as the pan DR epitope ("PADRE"). PADRE is described in US 5,736141, WO 95/07707, and Alexander J et al., Immunity, 1:751-761 (1994). A preferred PADRE peptide is **AK**XVAAW**TL**KAAA (SEQ ID NO:11), (common residues bolded) wherein X is preferably cyclohexylalanine tyrosine or phenylalanine, with cyclohexylalanine being most preferred.

Immunogenic agents can be linked to carriers by chemical crosslinking. Techniques for linking an immunogen to a carrier include the formation of disulfide linkages using N-succinimidyl-3-(2-pyridyl-thio) propionate (SPDP) and succinimidyl 4-(N-maleimidomethyl)cyclohexane-1-carboxylate (SMCC) (if the peptide lacks a sulfhydryl group, this can be provided by addition of a cysteine residue). These reagents create a disulfide linkage between themselves and peptide cysteine resides on one protein and an amide linkage through the epsilon-amino on a lysine, or other free amino group in other amino acids. A variety of such disulfide/amide-forming agents are described by Immun. Rev. 62, 185 (1982). Other bifunctional coupling agents form a thioether rather than a disulfide linkage. Many of these thio-ether-forming agents are commercially available and include reactive esters of 6-maleimidocaproic acid, 2-bromoacetic acid, and 2-iodoacetic acid, 4-(N-maleimido-methyl)cyclohexane-1-carboxylic acid. The carboxyl groups can be activated by combining them with succinimide or 1-hydroxyl-2-nitro-4-sulfonic acid, sodium salt.

Immunogenicity can be improved through the addition of spacer residues (e.g., Gly-Gly) between the Tₕ epitope and the peptide immunogen herein disclosed. In addition to physically separating the Tₕ epitope from the B cell epitope (*i.e.,* the peptide immunogen), the glycine residues can disrupt any artificial secondary structures created by the joining of the Tₕ epitope with the peptide immunogen, and thereby eliminate interference between the T and/or B cell responses. The conformational separation between the helper epitope and the antibody eliciting domain thus permits more efficient interactions between the presented immunogen and the appropriate Tₕ and B cells.

To enhance the induction of T cell immunity in a large percentage of subjects displaying various HLA types to an agent of the present invention, a mixture of conjugates with different Tₕ cell epitopes can be prepared. The mixture may contain a mixture of at least two conjugates with different Tₕ cell epitopes, a mixture of at least three conjugates with different Tₕ cell epitopes, or a mixture of at least four conjugates with different Tₕ cell epitopes. The mixture may be administered with an adjuvant.

Immunogenic peptides can also be expressed as fusion proteins with carriers (*i.e.,* heterologous peptides). The immunogenic peptide can be linked at its amino terminus, its carboxyl terminus, or both to a carrier. Optionally, multiple repeats of the immunogenic peptide can be present in the fusion protein. Optionally, an immunogenic peptide can be linked to multiple copies of a heterologous peptide, for example, at both the N and C termini of the peptide. Optionally, multiple copies of an immunogenic peptide can be linked to multiple copies of a heterologous peptide. which are linked to each other. Some carrier peptides serve to induce a helper T-cell response against the carrier peptide. The induced helper T-cells in turn induce a B-cell response against the immunogenic peptide linked to the carrier.

Some examples of fusion proteins suitable for use herein disclosed are shown below. Some of these fusion proteins comprise segments of Aβ linked to tetanus toxoid epitopes such as described in US 5,196,512, EP 378,881 and EP 427,347. Some fusion proteins comprise segments of Aβ linked to at least one PADRE peptide described in US 5,736,142. Some heterologous peptides are promiscuous T-cell epitopes, while other heterologous peptides are universal T-cell epitopes. In some methods, the agent for administration is simply a single fusion protein with an Aβ segment linked to a heterologous segment in linear configuration. The therapeutic agents herein diclosed can be represented using a formula. For example, in some methods, the agent is multimer of fusion proteins represented by the formula 2^{x}, in which x is an integer from 1-5. Preferably x is 1, 2 or 3, with 2 being most preferred. When x is two, such a multimer has four fusion proteins linked in a preferred configuration referred to as MAP4 (see US 5,229,490).

The MAP4 configuration is shown below, where branched structures are produced by initiating peptide synthesis at both the N terminal and side chain amines of lysine. Depending upon the number of times lysine is incorporated into the sequence and allowed to branch, the resulting structure will present multiple N termini. In this example, four identical N termini have been produced on the branched lysine-containing core. Such multiplicity greatly enhances the responsiveness of cognate B cells. In the examples below, Z refers to an immunogenic fragment of Aβ, and Z1-4 refer to immunogenic fragment(s) of Aβ. The fragments can be the same as each other or different.

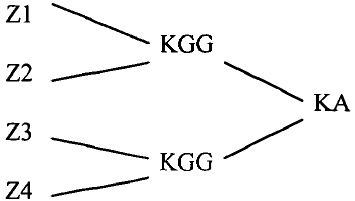

Other examples of fusion proteins include:
Z-Tetanus toxoid 830-844 in a MAP4 configuration:
   Z-QYIKANSKFIGITEL (SEQ ID NO:12)
Z-Tetanus toxoid 947-967 in a MAP4 configuration:
   Z-FNNFTVSFWLRVPKVSASHLE (SEQ ID NO: 13)
Z-Tetanus toxoid 830-844 in a MAP4 configuration:
   Z-QYIKANSKFIGITEL (SEQ ID NO:14)
Z-Tetanus toxoid 830-844 + 947-967 in a linear configuration:
   Z-QYIKANSKFIGITELFNNFTVSFWLRVPKVSASHLE (SEQ ID NO:15)

PADRE peptide (all in linear configurations), wherein X is preferably cyclohexylalanine, tyrosine or phenylalanine, with cyclohexylalanine being most preferred-Z: AKXVAAWTLKAAA-Z (SEQ ID NO:16)
**Z x 3**-PADRE peptide:
   **Z-Z-Z**-AKXVAAWTLKAAA (SEQ ID NO: 17)
**Z** - ovalbumin 323-339 in a linear configuration:
   **Z**-ISQAVHAAHAEINEAGR (SEQ ID NO:20)

Further examples of fusion proteins include:
AKXVAAWTLKAAA-**Z-Z-Z-Z** (SEQ ID NO:18)
**Z**-AKXVAAWTLKAAA (SEQ ID NO: 19)
PKYVKQNTLKLAT-**Z-Z-Z** (SEQ ID NO:21)
**Z**-PKYVKQNTLKLAT-**Z** (SEQ ID NO:22)
**Z-Z-Z**-PKYVKQNTLKLAT (SEQ ID NO:23)
**Z-Z**-PKYVKQNTLKLAT (SEQ ID NO:24)
**Z**-PKYVKQNTLKLAT-EKKIAKMEKASSVFNV-QYIKANSKFIGITEL-FNNFTVSFWLRVPKVSASHLE-Z-**Z-Z-Z**-QYIKANSKFIGITEL-FNNFTVSFWLRVPKVSASHLE (SEQ ID NO:25) **Z**-QYIKANSKFIGITELCFNNFTVSFWLRVPKVSASHLE-**Z-**QYIKANSKFIGITELCFNNFTVSFWLRVPKVSASHLE-**Z** (SEQ ID NO:26)
Z-QYIKANSKFIGITEL (SEQ ID NO:27) on a 2 branched resin: fragments can be the same as each other or different.

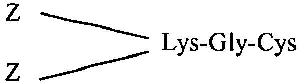

The same or similar carrier proteins and methods of linkage can be used for generating immunogens to be used in generation of antibodies against Aβ or an immunogenic fragment of Aβ. For example, Aβ or an immunogenic fragment of Aβ linked to a carrier can be administered to a laboratory animal in the production of monoclonal antibodies to Aβ or an immunogenic fragment of Aβ.

### V. Nucleic Acid Encoding Therapeutic Agents

Immune responses against amyloid deposits can also be induced by administration of nucleic acids encoding segments of Aβ peptide, and fragments thereof, other peptide immunogens, or antibodies and their component chains used for passive immunization. Such nucleic acids can be DNA or RNA. A nucleic acid segment encoding an immunogen is typically linked to regulatory elements, such as a promoter and enhancer, that allow expression of the DNA segment in the intended target cells of a patient. For expression in blood cells, as is desirable for induction of an immune response, promoter and enhancer elements from light or heavy chain immunoglobulin genes or the CMV major intermediate early promoter and enhancer are suitable to direct expression. The linked regulatory elements and coding sequences are often cloned into a vector. For administration of double-chain antibodies, the two chains can be cloned in the same or separate vectors. The nucleic acids encoding therapeutic agents herein disclosed can also encode at least one T cell epitope. The disclosures herein which relate to the use of adjuvants and the use of carriers apply *mutatis mutandis* to their use with the nucleic acids encoding the therapeutic agents herein disclosed.

A number of viral vector systems are available including retroviral systems *(see, e.g.,* Lawrie and Tumin, Cur. Opin. Genet. Develop. 3, 102-109 (1993)); adenoviral vectors (*see, e.g.,* Bett et al., J. Virol. 67, 5911 (1993)); adeno-associated virus vectors (see, *e.g.,* Zhou et al., J. Exp. Med. 179, 1867 (1994)), viral vectors from the pox family including vaccinia virus and the avian pox viruses, viral vectors from the alpha virus genus such as those derived from Sindbis and Semliki Forest Viruses (see, *e.g.,* Dubensky et al., J. Virol. 70, 508-519 (1996)), Venezuelan equine encephalitis virus (see US 5,643,576) and rhabdoviruses, such as vesicular stomatitis virus (see WO 96/34625)and papillomaviruses (Ohe et al., Human Gene Therapy 6, 325-333 (1995); Woo et al., WO 94/12629 and Xiao & Brandsma, Nucleic Acids. Res. 24, 2630-2622 (1996)).

DNA encoding an immunogen, or a vector containing the same, can be packaged into liposomes. Suitable lipids and related analogs are described by US 5,208,036, 5,264,618, 5,279,833 and 5,283,185. Vectors and DNA encoding an immunogen can also be adsorbed to or associated with particulate carriers, examples of which include polymethyl methacrylate polymers and polylactides and poly(lactide-co-glycolides), see, *e.g*., McGee et al., J. Micro Encap. (1996).

Gene therapy vectors or naked DNA can be delivered in vivo by administration to an individual patient, typically by systemic administration (e.g., intravenous, intraperitoneal, nasal, gastric, intradermal, intramuscular, subdermal, or intracranial infusion) or topical application (*see e.g.,* US 5,399,346). Such vectors can further include facilitating agents such as bupivacine (US 5,593,970). DNA can also be administered using a gene gun. (See Xiao & Brandsma, *supra*.) The DNA encoding an immunogen is precipitated onto the surface of microscopic metal beads. The microprojectiles are accelerated with a shock wave or expanding helium gas, and penetrate tissues to a depth of several cell layers. For example, The Accel™ Gene Delivery Device manufactured by Agacetus, Inc. Middleton WI is suitable. Alternatively, naked DNA can pass through skin into the blood stream simply by spotting the DNA onto skin with chemical or mechanical irritation (see WO 95/05853).

Also disclosed herein, vectors encoding immunogens can be delivered to cells ex vivo, such as cells explanted from an individual patient (e.g., lymphocytes, bone marrow aspirates, tissue biopsy) or universal donor hematopoietic stem cells, followed by reimplantation of the cells into a patient, usually after selection for cells which have incorporated the vector.

### VI. Adjuvants

Immunogenic agents of the invention, such as peptides, are sometimes administered in combination with an adjuvant. The adjuvant increases the titer of induced antibodies and/or the binding affinity of induced antibodies relative to the situation if the peptide were used alone. A variety of adjuvants can be used in combination with an immunogenic fragment of Aβ, to elicit an immune response. Preferred adjuvants augment the intrinsic response to an immunogen without causing conformational changes in the immunogen that affect the qualitative form of the response. Preferred adjuvants include aluminum hydroxide and aluminum phosphate, 3 De-O-acylated monophosphoryl lipid A (MPL™) (*see* GB 2220211 (RIBI ImmunoChem Research Inc., Hamilton, Montana, now part of Corixa). Stimulon™ QS-21 is a triterpene glycoside or saponin isolated from the bark of the Quillaja Saponaria Molina tree found in South America (*see* Kensil et al., in Vaccine Design: The Subunit and Adjuvant Approach (eds. Powell & Newman, Plenum Press, NY, 1995); US Patent No. 5,057,540), (Aquila BioPharmaceuticals, Framingham, MA). Other adjuvants are oil in water emulsions (such as squalene or peanut oil), optionally in combination with immune stimulants, such as monophosphoryl lipid A (see Stoute et al., N. Engl. J. Med. 336, 86-91 (1997)), pluronic polymers, and killed mycobacteria. Another adjuvant is CpG (WO 98/40100). Adjuvants can be administered as a component of a therapeutic composition with an active agent or can be administered separately, before, concurrently with, or after administration of the therapeutic agent.

A preferred class of adjuvants is aluminum salts (alum), such as alum hydroxide, alum phosphate, alum sulfate. Such adjuvants can be used with or without other specific immunostimulating agents such as MPL or 3-DMP, QS-21, polymeric or monomeric amino acids such as polyglutamic acid or polylysine. Another class of adjuvants is oil-in-water emulsion formulations. Such adjuvants can be used with or without other specific immunostimulating agents such as muramyl peptides (e.g., N-acetylmuramyl-L-threonyl-D-isoglutamine (thr-MDP), N-acetyl-normuramyl-L-alanyl-D-isoglutamine (nor-MDP), N-acetylmuramyl-L-alanyl-D-isoglutaminyl-L-alanine-2-(1'-2'dipalmitoyl-sn-glycero-3-hydroxyphosphoryloxy)-ethylamine (MTP-PE), N-acetylglucsaminyl-N-acetylmuramyl-L-Al-D-isoglu-L-Ala-dipalmitoxy propylamide (DTP-DPP) theramideTM), or other bacterial cell wall components. Oil-in-water emulsions include (a) MF59 (WO 90/14837), containing 5% Squalene, 0.5% Tween 80, and 0.5% Span 85 (optionally containing various amounts of MTP-PE) formulated into submicron particles using a microfluidizer such as Model 110Y microfluidizer (Microfluidics, Newton MA), (b) SAF, containing 10% Squalene, 0.4% Tween 80, 5% pluronic-blocked polymer L121, and thr-MDP, either microfluidized into a submicron emulsion or vortexed to generate a larger particle size emulsion, and (c) Ribi™ adjuvant system (RAS), (Ribi ImmunoChem, Hamilton, MT) containing 2% squalene, 0.2% Tween 80, and one or more bacterial cell wall components from the group consisting of monophosphoryllipid A (MPL), trehalose dimycolate (TDM), and cell wall skeleton (CWS), preferably MPL + CWS (Detox™).

Another class of preferred adjuvants is saponin adjuvants, such as Stimulon™ (QS-21, Aquila, Framingham, MA) or particles generated therefrom such as ISCOMs (immunostimulating complexes) and ISCOMATRIX. Other adjuvants include RC-529, GM-CSF and Complete Freund's Adjuvant (CFA) and Incomplete Freund's Adjuvant (IFA). Other adjuvants include cytokines, such as interleukins (e.g., IL-1 a and β peptides,, IL-2, IL-4, IL-6, IL-12, IL13, and IL-15), macrophage colony stimulating factor (M-CSF), granulocyte-macrophage colony stimulating factor (GM-CSF), tumor necrosis factor (TNF), chemokines, such as MIP1α and β and RANTES. Another class of adjuvants is glycolipid analogues including N-glycosylamides, N-glycosylureas and N-glycosylcarbamates, each of which is substituted in the sugar residue by an amino acid, as immuno-modulators or adjuvants (*see* US Pat. No. 4,855,283). Heat shock proteins, *e.g.,* HSP70 and HSP90, may also be used as adjuvants.

An adjuvant can be administered with an immunogen as a single composition, or can be administered before, concurrent with or after administration of the immunogen. Immunogen and adjuvant can be packaged and supplied in the same vial or can be packaged in separate vials and mixed before use. Immunogen and adjuvant are typically packaged with a label indicating the intended therapeutic application. If immunogen and adjuvant are packaged separately, the packaging typically includes instructions for mixing before use. The choice of an adjuvant and/or carrier depends on the stability of the immunogenic formulation containing the adjuvant, the route of administration, the dosing schedule, the efficacy of the adjuvant for the species being vaccinated, and, in humans, a pharmaceutically acceptable adjuvant is one that has been approved or is approvable for human administration by pertinent regulatory bodies. For example, Complete Freund's adjuvant is not suitable for human administration. Alum, MPL and QS-21 are preferred. Optionally, two or more different adjuvants can be used simultaneously. Preferred combinations include alum with MPL, alum with QS-21, MPL with QS-21, MPL or RC-529 with GM-CSF, and alum, QS-21 and MPL together. Also, Incomplete Freund's adjuvant can be used (Chang et al., Advanced Drug Delivery Reviews 32, 173-186 (1998)), optionally in combination with any of alum, QS-21, and MPL and all combinations thereof.

### VII. Passive Administration Of Antibodies

Active immunization with fragments of Aβ can be combined with passive administration of antibodies. The antibodies used for passive administration can be antibodies to N-terminal epitopes of Aβ for induction of a phagocytic clearing response of plaques, or can be antibodies to central or C-terminal regions of Aβ for clearing soluble Aβ. In some methods, passive administration with an antibody to an N-terminal region antibody is performed first to clear existing amyloid deposits. Subsequently, a fragment from a central or C-terminal region of Aβ is administered to prevent further deposition of amyloid deposits from soluble Aβ. In other method, active administration with a fragment to a central or C-terminal portion of Aβ is performed first to generate antibodies that clear soluble Aβ. Then when the level of antibodies in the blood starts to wane, an additional dose is supplied by passive administration of antibodies that specifically bind to a central or C-terminal epitope of Aβ.

Antibodies suitable for use in passive administration are described in WO 00/72880 and WO 02/46237. Preferred antibodies specifically binding to an N-terminal epitope of Aβ bind to an epitope starting at resides 1-3 and ending at residues 7-11 of Aβ. Some preferred antibodies specifically bind to epitopes within amino acids 1-3, 1-4, 1-5, 1-6, 1-7 or 3-7. Some preferred antibodies specifically bind to an epitope starting at resides 1-3 and ending at residues 7-11 of Aβ. Such antibodies typically specifically bind to amyloid deposits but may or may not bind to soluble Aβ. Some preferred antibodies specifically binding to a C-terminal epitope of Aβ specifically bind to a naturally occurring long form of Aβ (*i.e.,* Aβ42 and Aβ43 without specifically binding to a naturally occurring short form of Aβ *(i.e.,* Aβ39, 40 or 41). Antibodies to C-terminal and central epitopes of typically specifically bind to soluble without specific binding to amyloid deposits. When an antibody is said to specifically bind to an epitope within specified residues, such as Aβ 1-5 for example, what is meant is that the antibody specifically binds to a polypeptide containing the specified residues *(i.e.,* Aβ 1-5 in this an example). Such an antibody does not necessarily contact every residue within Aβ 1-5. Nor does every single amino acid substitution or deletion with in Aβ1-5 necessarily significantly affect binding affinity. Epitope specificity of an antibody can be determined, for example, as described by WO 00/72880.

Antibodies can be polyclonal or monoclonal. Polyclonal sera typically contain mixed populations of antibodies specifically binding to several epitopes along the length of Aβ. However, polyclonal sera can be specific to a particular segment of Aβ, such as Aβ1-10. Preferred antibodies are chimeric, humanized (see Queen et al., Proc. Natl. Acad. Sci. USA 86:10029-10033 (1989) and WO 90/07861, US 5,693,762, US 5,693,761, US 5,585,089, US 5,530,101 and Winter, US 5,225,539), or human (Lonberg et al., WO93/12227 (1993); US 5,877,397, US 5,874,299, US 5,814,318, US 5,789,650, US 5,770,429, US 5,661,016, US 5,633,425, US 5,625,126, US 5,569,825, US 5,545,806, Nature 148, 1547-1553 (1994), Nature Biotechnology 14, 826 (1996), Kucherlapati, WO 91/10741 (1991)). Several mouse antibodies of different binding specificities are available as starting materials for making humanized antibodies. Human isotype IgG1 is preferred for antibodies to the N-terminal region of because of it having highest affinity of human isotypes for the FcRI receptor on phagocytic cells. Some antibodies specifically bind to Aβ with a binding affinity greater than or equal to about 10⁷, 10⁸, 10⁹, or 10¹⁰ M⁻¹.

### VIII. Patients Amenable To Treatment

Patients amenable to treatment include individuals at risk of disease but not showing symptoms, as well as patients presently showing symptoms. In the case of Alzheimer's disease, virtually anyone is at risk of suffering from Alzheimer's disease if he or she lives long enough. Therefore, the present methods can be administered prophylactically to the general population without the need for any assessment of the risk of the subject patient. The present methods are especially useful for individuals who do have a known genetic risk of Alzheimer's disease. Such individuals include those having relatives who have experienced this disease, and those whose risk is determined by analysis of genetic or biochemical markers. Genetic markers of risk toward Alzheimer's disease include mutations in the APP gene, particularly mutations at position 717 and positions 670 and 671 referred to as the Hardy and Swedish mutations respectively (see Hardy, TINS, supra). Other markers of risk are mutations in the presenilin genes, PS1 and PS2, and ApoE4, family history of AD, hypercholesterolemia or atherosclerosis. Individuals presently suffering from Alzheimer's disease can be recognized from characteristic dementia, as well as the presence of risk factors described above. In addition, a number of diagnostic tests are available for identifying individuals who have AD. These include measurement of CSF tau and Aβ42 levels. Elevated tau and decreased Aβ42 levels signify the presence of AD. Individuals suffering from Alzheimer's disease can also be diagnosed by ADRDA criteria as discussed in WO 00/72880.

In asymptomatic patients, treatment can begin at any age *(e.g.,* 10, 20, 30). Usually, however, it is not necessary to begin treatment until a patient reaches 40, 50, 60 or 70. Treatment typically entails multiple dosages over a period of time. Treatment can be monitored by assaying antibody, or activated T-cell (a side effect) or B-cell responses to the therapeutic agent (e.g., Aβ peptide) over time. If the response falls, a booster dosage is indicated. In the case of potential Down's syndrome patients, treatment can begin antenatally by administering therapeutic agent to the mother or shortly after birth.

### IX. Treatment Regimes

In general treatment regimes involve administering an agent effective to induce an immunogenic response to Aβ, preferably an immunogenic fragment of Aβ to a patient. In prophylactic applications, pharmaceutical compositions or medicaments are administered to a patient susceptible to, or otherwise at risk of, Alzheimer's disease in an amount sufficient to eliminate or reduce the risk, lessen the severity, or delay the onset of the disease, including physiological, biochemical, histologic and/or behavioral symptoms of the disease, its complications and intermediate pathological phenotypes presenting during development of the disease. In therapeutic applications, an agent is administered to a patient suspected of, or already suffering from such a disease in a regime comprising an amount and frequency of administration of the agent sufficient to cure, or at least partially arrest, or inhibit deterioration of the symptoms of the disease (physiological, biochemical, histologic and/or behavioral), including its complications and intermediate pathological phenotypes in development of the disease. In some methods, administration of agent reduces or eliminates myocognitive impairment in patients that have not yet developed characteristic Alzheimer's pathology. An amount adequate to accomplish therapeutic or prophylactic treatment is defined as a therapeutically- or prophylactically-effective dose. A combination of amount and dosage frequency adequate to accomplish the therapeutic or prophylactic treatment is defined as a therapeutically- or prophylactically-effective regime. In both prophylactic and therapeutic regimes, agents are usually administered in several dosages until a sufficient immune response has been achieved. A dosage and frequency of administrations adequate to accomplish therapeutic or prophylactic treatment is defined as a therapeutically- or prophylactically-effective regime. Typically, the patient's immune response is monitored and repeated dosages are given if the immune response starts to wane. The immune response can be monitored by detecting antibodies to AB in the blood in the patient, detecting levels of AB or plaques in the brain or symptoms by a psychometric measure, such as the MMSE, and the ADAS, which is a comprehensive scale for evaluating patients with Alzheimer's Disease status and function.

Effective doses of the agents and compositions herein disclosed for the treatment of the above described conditions vary depending upon many different factors, including means of administration, target site, physiological state of the patient, whether the patient is human or an animal, other medications administered, and whether treatment is prophylactic or therapeutic. Usually, the patient is a human but nonhuman mammals including transgenic mammals can also be treated. Treatment dosages need to be titrated to optimize safety and efficacy. The amount of immunogen depends on whether adjuvant is also administered, with higher dosages being required in the absence of adjuvant. The amount of an immunogen for administration sometimes varies from 1-500 µg per patient and more usually from 5-500 µg per injection for human administration. Occasionally, a higher dose of 1-2 mg per injection is used. Typically at least 10, 20, 50 or 100 µg is used for each human injection. The mass of immunogen also depends on the mass ratio of immunogenic epitope within the immunogen to the mass of immunogen as a whole. Typically, 10⁻³ to 10⁻⁵ micromoles of immunogenic epitope are used for microgram of immunogen. The timing of injections can vary significantly from once a day, to once a year, to once a decade. On any given day that a dosage of immunogen is given, the dosage is greater than 1 µg/patient and usually greater than 10 µg/ patient if adjuvant is also administered, and greater than 10 µg/patient and usually greater than 100 µg/patient in the absence of adjuvant. A typical regimen consists of an immunization followed by booster injections at time intervals, such as 6 week intervals. Another regimen consists of an immunization followed by booster injections 1, 2 and 12 months later. Another regimen entails an injection every two months for life. Alternatively, booster injections can be on an irregular basis as indicated by monitoring of immune response.

Disclosed herein are doses for nucleic acids encoding immunogens range from about 10 ng to 1 g, 100 ng to 100 mg, 1 µg to 10 mg, or 30-300 µg DNA per patient. Doses for infectious viral vectors vary from 10-100, or more, virions per dose.

For passive immunization with an antibody (in combination therapies), the dosage ranges from about 0.0001 to 100 mg/kg, and more usually 0.01 to 5 mg/kg, of the host body weight. For example dosages can be 1 mg/kg body weight or 10 mg/kg body weight or within the range of 1-10 mg/kg or in other words, 70 mg or 700 mg or within the range of 70-700 mg, respectively, for a 70 kg patient. An exemplary treatment regime entails administration once per every two weeks or once a month or once every 3 to 6 months. In some methods, two or more monoclonal antibodies with different binding specificities are administered simultaneously, in which case the dosage of each antibody administered falls within the ranges indicated. Antibody is usually administered on multiple occasions. Intervals between single dosages can be weekly, monthly or yearly. Intervals can also be irregular as indicated by measuring blood levels of antibody to Aβ in the patient. In some methods, dosage is adjusted to achieve a plasma antibody concentration of 1-1000 µg/ml and in some methods 25-300 µg/ml. Alternatively, antibody can be administered as a sustained release formulation, in which case less frequent administration is required. Dosage and frequency vary depending on the half-life of the antibody in the patient. In general, human antibodies show the longest half life, followed by humanized antibodies, chimeric antibodies, and nonhuman antibodies. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patent can be administered a prophylactic regime.

Agents for inducing an immune response can be administered by parenteral, topical, intravenous, oral, subcutaneous, intraarterial, intracranial, intraperitoneal, intranasal or intramuscular means for prophylactic and/or therapeutic treatment. The most typical route of administration of an immunogenic agent is subcutaneous although other routes can be equally effective. The next most common route is intramuscular injection. This type of injection is most typically performed in the arm or leg muscles. In some methods, agents are injected directly into a particular tissue where deposits have accumulated, *e.g*., intracranial injection. Intramuscular injection or intravenous infusion are preferred for administration of antibody (in combination therapies). In some methods, particular therapeutic antibodies are injected directly into the cranium. In some methods, antibodies are administered as a sustained release composition or device, such as a Medipad™ device.

Agents herein disclosed are often administered as pharmaceutical compositions comprising an active therapeutic agent, *i.e.*, and a variety of other pharmaceutically acceptable components. See Remington's Pharmaceutical Science (15th ed., Mack Publishing Company, Easton, Pennsylvania, 1980). The preferred form depends on the intended mode of administration and therapeutic application. The compositions can also include, depending on the formulation desired, pharmaceutically-acceptable, non-toxic carriers or diluents, which are defined as vehicles commonly used to formulate pharmaceutical compositions for animal or human administration. The diluent is selected so as not to affect the biological activity of the combination. Examples of such diluents are distilled water, physiological phosphate-buffered saline, Ringer's solutions, dextrose solution, and Hank's solution. In addition, the pharmaceutical composition or formulation may also include other carriers, adjuvants, or nontoxic, nontherapeutic, nonimmunogenic stabilizers and the like.

Pharmaceutical compositions can also include large, slowly metabolized macromolecules such as proteins, polysaccharides such as chitosan, polylactic acids, polyglycolic acids and copolymers (such as latex functionalized sepharose(TM), agarose, cellulose, and the like), polymeric amino acids, amino acid copolymers, and lipid aggregates (such as oil droplets or liposomes). Additionally, these carriers can function as immunostimulating agents *(i.e.,* adjuvants).

For parenteral administration, agents herein disclosed can be administered as injectable dosages of a solution or suspension of the substance in a physiologically acceptable diluent with a pharmaceutical carrier that can be a sterile liquid such as water oils, saline, glycerol, or ethanol. Additionally, auxiliary substances, such as wetting or emulsifying agents, surfactants, pH buffering substances and the like can be present in compositions. Other components of pharmaceutical compositions are those of petroleum, animal, vegetable, or synthetic origin, for example, peanut oil, soybean oil, and mineral oil. In general, glycols such as propylene glycol or polyethylene glycol are preferred liquid carriers, particularly for injectable solutions. Antibodies can be administered in the form of a depot injection or implant preparation which can be formulated in such a manner as to permit a sustained release of the active ingredient. An exemplary composition comprises monoclonal antibody at 5 mg/mL, formulated in aqueous buffer consisting of 50 mM L-histidine, 150 mM NaCl, adjusted to pH 6.0 with HCl. Composition for parenteral administration are typically substantially sterile, isotonic and manufactured under GMP conditions of the FDA or similar body.

Typically, compositions are prepared as injectables, either as liquid solutions or suspensions; solid forms suitable for solution in, or suspension in, liquid vehicles prior to injection can also be prepared. The preparation also can be emulsified or encapsulated in liposomes or micro particles such as polylactide, polyglycolide, or copolymer for enhanced adjuvant effect, as discussed above (see Langer, Science 249, 1527 (1990) and Hanes Advanced Drug Delivery Reviews 28, 97-119 (1997). The agents herein disclosed can be administered in the form of a depot injection or implant preparation which can be formulated in such a manner as to permit a sustained or pulsatile release of the active ingredient.

Additional formulations suitable for other modes of administration include oral, intranasal, and pulmonary formulations, suppositories, and transdermal applications.

For suppositories, binders and carriers include, for example, polyalkylene glycols or triglycerides; such suppositories can be formed from mixtures containing the active ingredient in the range of 0.5% to 10%, preferably 1%-2%. Oral formulations include excipients, such as pharmaceutical grades of mannitol, lactose, starch, magnesium stearate, sodium saccharine, cellulose, and magnesium carbonate. These compositions take the form of solutions, suspensions, tablets, pills, capsules, sustained release formulations or powders and contain 10%-95% of active ingredient, preferably 25%-70%.

Topical application can result in transdermal or intradermal delivery. Topical administration can be facilitated by co-administration of the agent with cholera toxin or detoxified derivatives or subunits thereof or other similar bacterial toxins (See Glenn et al., Nature 391, 851 (1998)). Co-administration can be achieved by using the components as a mixture or as linked molecules obtained by chemical crosslinking or expression as a fusion protein.

Alternatively, transdermal delivery can be achieved using a skin path or using transferosomes (Paul et al., Eur. J. Immunol. 25, 3521-24 (1995); Cevc et al., Biochem. Biophys. Acta 1368, 201-15 (1998)).

### X. Methods of Monitoring

The disclosure provides methods of detecting an antibody response against Aβ peptide in a patient suffering from or susceptible to an amyloidogenic disease. The methods are particularly useful for monitoring a course of treatment being administered to a patient. The methods can be used to monitor both therapeutic treatment on symptomatic patients and prophylactic treatment on asymptomatic patients. Some methods entail determining a baseline value of an antibody response in a patient before administering a dosage of an immunogenic agent, and comparing this with a value for the immune response after treatment. A significant increase *(i.e.,* greater than the typical margin of experimental error in repeat measurements of the same sample, expressed as one standard deviation from the mean of such measurements) in value of the antibody response signals a positive treatment outcome (*i.e.,* that administration of the agent has achieved or augmented an immune response). If the value for the antibody response does not change significantly, or decreases, a negative treatment outcome is indicated. In general, patients undergoing an initial course of treatment with an immunogenic agent are expected to show an increase in antibody response with successive dosages, which eventually reaches a plateau. Administration of agent is generally continued while the antibody response is increasing. Attainment of the plateau is an indicator that the administered of treatment can be discontinued or reduced in dosage or frequency.

In other methods, a control value *(i.e.,* a mean and standard deviation) of an antibody response is determined for a control population. Typically the individuals in the control population have not received prior treatment. Measured values of the antibody response in a patient after administering a therapeutic agent are then compared with the control value. A significant increase relative to the control value (*e.g*., greater than one standard deviation from the mean) signals a positive treatment outcome. A lack of significant increase or a decrease signals a negative treatment outcome. Administration of agent is generally continued while the antibody response is increasing relative to the control value. As before, attainment of a plateau relative to control values in an indicator that the administration of treatment can be discontinued or reduced in dosage or frequency.

In other methods, a control value of antibody response (*e.g*., a mean and standard deviation) is determined from a control population of individuals who have undergone treatment with a therapeutic agent and whose antibody responses have reached a plateau in response to treatment. Measured values of antibody response in a patient are compared with the control value. If the measured level in a patient is not significantly different (e.g., more than one standard deviation) from the control value, treatment can be discontinued. If the level in a patient is significantly below the control value, continued administration of agent is warranted. If the level in the patient persists below the control value, then a change in treatment regime, for example, use of a different adjuvant, fragment or switch to passive administration may be indicated.

In other methods, a patient who is not presently receiving treatment but has undergone a previous course of treatment is monitored for antibody response to determine whether a resumption of treatment is required. The measured value of antibody response in the patient can be compared with a value of antibody response previously achieved in the patient after a previous course of treatment. A significant decrease relative to the previous measurement *(i.e.,* greater than a typical margin of error in repeat measurements of the same sample) is an indication that treatment can be resumed. Alternatively, the value measured in a patient can be compared with a control value (mean plus standard deviation) determined in a population of patients after undergoing a course of treatment. Alternatively, the measured value in a patient can be compared with a control value in populations of prophylactically treated patients who remain free of symptoms of disease, or populations of therapeutically treated patients who show amelioration of disease characteristics. In all of these cases, a significant decrease relative to the control level *(i.e.,* more than a standard deviation) is an indicator that treatment should be resumed in a patient.

The tissue sample for analysis is typically blood, plasma, serum, mucous or cerebrospinal fluid from the patient. The sample is analyzed for indication of an immune response to any form of Aβ peptide, typically Aβ42 or the peptide used for immunization. The immune response can be determined from the presence of antibodies that specifically bind to Aβ peptide. Antibodies can be detected in a binding assay to a ligand that specifically binds to the antibodies. Typically the ligand is immobilized. Binding can be detected using a labeled anti-idiotypic antibody.

In combination regimes employing both active and passive administration, analogous approaches can be used to monitor levels of antibody resulting from passive administration as described in WO 00/72880.

### Examples

### Materials and Methods

**Aβ Fragments.** Peptides corresponding to Aβ1-5, Aβ3-9, Aβ5-11, Aβ15-24 and the reverse sequence Aβ5-1 were synthesized contiguous to a 17-amino acid T cell epitope derived from ovalbumin (amino acids 323-339 - ISQAVHAAHAEINEAGR (SEQ ID NO:3)) on a branched peptide framework (triple-lysine core with four peptide arms) to produce a multi-antigen peptide, as described by Tam, J. P. (1988) Proc. Natl. Acad. Sci. USA 85, 5409-5413. Polyclonal antibodies (Pab) Aβ1-42 were raised and the immunoglobulin fraction isolated, as previously described by Bard, F. et al., (2000) Nat. Med. 6, 916-919. Polyclonal Pab-EL16, Pab-EL17, and Pab-EL20 were obtained from the sera of PDAPP mice immunized with peptides corresponding respectively to Aβ1-7, Aβ15-24, and Aβ3-9 that had been synthesized on a branched framework, as described above. Pab-EL26 was obtained from the sera of mice immunized with Aβ(7-1)-42. The peptides were synthesized by AnaSpec, San Jose, CA, USA.

**Immunization Procedures.** 100 µg of Aβ fragment was administered by intraperitoneal injection in complete Freund's adjuvant, followed by boosts with 100 µg peptide in incomplete Freund's adjuvant at 2 and 4 weeks, and monthly thereafter.

**Antibody Binding to Aggregated and Soluble Aβ1-42.** Serum titers (determined by serial dilution) and monoclonal antibody binding to aggregated synthetic Aβ1-42 were performed by ELISA as previously described by Schenk D. et al., (1999) Nature 400, 173-177. Soluble Aβ1-42 refers to the synthetic Aβ1-42 peptide sonicated in dimethyl sulfoxide. Serial dilutions of antibody were incubated with 50,000 cpm of ¹²⁵I-Aβ1-42 overnight at room temperature. 50 µl of a slurry containing 75 mg/ml protein A sepharose (Amersham Biosciences, Uppsala, Sweden)/200 µg rabbit anti-mouse IgG (H+L) (Jackson ImmunoResearch, West Grove, PA, USA) was incubated with the diluted antibodies for 1 hr at room temperature, washed twice, and counted on a Wallac gamma counter (PerkinElmer Life Science, Grove, IL, USA). All steps were performed in radioimmunoassay buffer consisting of 10 mM Tris, 0.5 M NaCl, 1 mg/ml gelatin, and 0.5% Nonidet P-40, pH 8.0.

### Results

A series of peptides were compared for their ability to trigger an efficacious antibody response *in vivo.* Twelve to thirteen month old PDAPP mice were immunized with one of three N-terminal peptide fragments (Aβ1-5, Aβ3-9, or Aβ5-11) or a fragment derived from an internal region of the peptide (Aβ15-24) (Fig 1a). The internal peptide Aβ15-24 encompasses the epitope of antibody 266, which exhibits high affinity for soluble Aβ (Seubert et al., (1992) Nature 359, 325-327.), does not recognize plaques in sections of unfixed AD or PDAPP tissue. Thus, it was of interest to determine whether a polyclonal response directed against this peptide could produce antibodies capable of plaque recognition, or whether reactivity with soluble Aβ alone was sufficient to provide efficacy. In these studies, a peptide with reverse sequence, Aβ5-1, served as a negative control. The peptides were synthesized contiguous to a 17-amino acid T cell epitope derived from ovalbumin and were presented in an identical multivalent configuration (see Materials and Methods). All of the peptides (except Aβ5-1 reversemer) produced sera that recognized aggregated synthetic Aβ1-42 by ELISA, although Aβ-11 and Aβ15-24 produced significantly higher titers than Aβ1-5 (p<0.01 and p<0.05, respectively) (Fig. 1b). In contrast, only sera against the N-terminal peptides were able to recognize Aβ within plaques; antisera against Aβ15-24 did not bind plaques in spite of strong reactivity with the synthetic aggregated peptide (Fig. 1c). There were also differences between the serum groups in their ability to capture soluble Aβ (Fig. 2a). Less than 30% of the sera from mice immunized with Aβ1-5 or Aβ3-9 captured the soluble peptide (27% and 5% respectively). In contrast, sera from approximately half of the animals immunized with Aβ5-11, and all of those immunized with Aβ15-24, captured soluble Aβ1-42.

Because the degree of Aβ deposition can vary greatly as PDAPP mice age, the *in vivo* study was designed with at least 30 animals per group. Efficacy data are shown for individual mice and expressed as the percentage of either amyloid burden or neuritic dystrophy relative to the mean of the control (set at 100%). Immunization with each of the three N-terminal peptides significantly reduced amyloid burden (46-61%, p<0.002) (Fig. 2b). Furthermore, Aβ3-9 and Aβ5-11 significantly reduced neuritic pathology (34% and 41% respectively, p<0.05), (Fig 2c). Immunization with Aβ15-24 provided no protection against either amyloid burden or neuritic pathology. These results support plaque binding as one mechanism for antibody efficacy. They also indicate that capture of soluble Aβ is not required for reduction of neuritic pathology since the antibody response against Aβ3-9 provided strong plaque reactivity and the highest level of protection against neuronal dystrophy, yet exhibited the weakest capacity for recognition of soluble peptide. Antibodies that bind to soluble Aβ without binding to plaques may also have such activity if administered at higher titers or over longer periods of time. Antibodies that bind to soluble Aβ without binding to plaques can also be useful in preventing formation and/or further deposition of Aβ. The high titer of antibodies generated by immunization with Aβ15-24 indicates that his fragment and subfragments thereof are particularly useful for generating high titers of soluble antibodies for this purpose.

Although the foregoing invention has been described in detail for purposes of clarity of understanding, it will be obvious that certain modifications may be practiced within the scope of the appended claims. Unless otherwise apparent from the context, each element, feature or embodiment of the invention can be used in combination with any other.

## Claims

1. A medicament comprising a fragment of Aβ consisting of Aβ16-23 (KLVFFAED) in the natural human amino acid form; wherein the fragment is linked to a carrier molecule to form a conjugate which helps elicit an immune response against the fragment;
for effecting treatment or prophylaxis of a disease associated with amyloid deposits of Aβ in the brain of a patient, whereby the induced antibodies specifically bind to soluble Aβ in the patient thereby inhibiting formation of amyloid deposits of Aβ in the brain from the soluble Aβ and thereby effecting treatment or prophylaxis of the disease.

2. The medicament of claim 1, wherein treating or effecting prophylaxis of the disease comprises administering in combination with said medicament a fragment of Aβ that induces antibodies specifically binding to Aβ at one or more epitopes within Aβ1-11.

3. The medicament of claim 2, wherein the fragment of Aβ that induces antibodies specifically binding to Aβ at an epitope within Aβ1-11 is suitable for administration before the Aβ16-23 fragment.

4. The medicament of claim 1, wherein treating or effecting prophylaxis of the disease comprises administering in combination with said medicament an antibody that specifically binds to Aβ at an epitope with Aβ1-11.

5. The medicament of claim 4, wherein the antibody that specifically binds to Aβ at an epitope within Aβ1-11 is suitable for administration before the Aβ16-23 fragment.

6. The medicament of any of the preceding claims, wherein the disease is **characterized by** cognitive impairment.

7. The medicament of any of the preceding claims, wherein the disease is selected from the group consisting of Alzheimer's disease, Down's syndrome and mild cognitive impairment.

8. The medicament of any of the preceding claims, wherein treating or effecting prophylaxis of the disease comprises monitoring the induced antibodies in the patient.

9. The medicament of any of the preceding claims, wherein the patient is asymptomatic.

10. The medicament of any of the preceding claims, wherein the patient is symptomatic and the medicament inhibits deterioration of the patient's symptoms.

11. The medicament of any of the preceding claims, wherein the patient is under 50 years of age.

12. The medicament of any of the preceding claims, wherein the patient has an inherited risk factor indicating susceptibility to Alzheimer's disease.

13. The medicament of claim 9, wherein the patient does not develop detectable symptoms for five years after the treatment or prophylaxis is first performed.

14. The medicament of any of claims 1-11 and 13, wherein the patient has no known risk factors for Alzheimer's disease.

15. The medicament of any of the preceding claims, which is suitable for administration at a dosage of at least 50 micrograms of the fragment on a plurality of days.

16. The medicament of any of the preceding claims, which is suitable for administration in multiple dosages over a period of at least three months.

17. The medicament of claim 16, wherein the dosages are at least 50 micrograms.

18. The medicament of any of the preceding claims, which further comprises an adjuvant that increases the level of antibodies induced by the fragment.

19. The medicament of any of the preceding claims, which is suitable for intraperitoneal, oral, intranasal, subcutaneous, intramuscular, topical or intravenous administration.

20. The medicament of any of the preceding claims, wherein treating or effecting prophylaxis of the disease further comprises monitoring the patient for level of induced antibodies in the blood of the patient.

21. The medicament of claim 1, wherein the carrier is a heterologous polypeptide.

22. The medicament of claim 1, wherein multiple copies of the fragment are linked to a carrier molecule to form a conjugate.

23. The medicament of claim 1, wherein multiple copies of the fragment are linked to multiple copies of the carrier molecule, which are linked to each other.

24. The medicament of claim 21, wherein the heterologous polypeptide comprises QYIKANSKFIGITEL (SEQ ID NO: 8).

25. The medicament of claim 21, wherein the heterologous polypeptide comprises the amino acid sequence AKXVAAWTLKAAA (SEQ ID NO: 11).

26. The medicament of claim 21, wherein the polypeptide induces a T-cell response against the heterologous polypeptide and thereby a B-cell response against the fragment.

27. The medicament of claim 1, wherein treating or effecting prophylaxis further comprises administering an adjuvant that enhances the titer and/or binding affinity of the induced antibodies relative to the titer and/or binding affinity of the antibodies induced by the fragment alone.

28. The medicament of claim 27, which comprises the adjuvant and the fragment.

29. The medicament of claim 27, wherein the adjuvant is suitable for administration before the fragment.

30. The medicament of claim 27, wherein the adjuvant is suitable for administration after the fragment.

31. The medicament of claim 27 wherein the adjuvant is selected from the group consisting of alum, MPL, QS-21 and incomplete Freund's adjuvant.

32. The medicament of any of the preceding claims, which comprises the fragment at a dosage of greater than 10 micrograms.

## Patentansprüche

1. Medikament, umfassend ein Fragment von Aβ, bestehend aus Aβ16-23 (KLVFFAED) in der natürlichen menschlichen Aminosäureform, wobei das Fragment mit einem Trägermolekül unter Bildung eines Konjugats verknüpft ist, dass das Hervorrufen einer Immunreaktion gegen das Fragment unterstützt,
zum Durchführen einer Behandlung oder Prophylaxe von einer Krankheit, die mit Amyloidablagerungen von Aβ im Hirn eines Patienten assoziiert ist, wobei die induzierten Antikörper spezifisch an lösliches Aβ in dem Patienten binden, wodurch die Bildung von Amyloidablagerungen von Aβ im Hirn von dem löslichen Aβ gehemmt werden und wodurch eine Behandlung oder Prophylaxe der Krankheit erfolgt.

2. Medikament nach Anspruch 1, wobei man beim Behandeln oder Durchführen einer Prophylaxe der Krankheit in Kombination mit dem genannten Medikament ein Fragment von Aβ verabreicht, das Antikörper induziert, die an einem oder mehreren Epitopen innerhalb von Aβ1-11 spezifisch an Aβ binden.

3. Medikament nach Anspruch 2, wobei das Fragment von Aβ, das Antikörper induziert, die an einem Epitop innerhalb von Aβ 1-11 spezifisch an Aβ binden, für die Verabreichung vor dem Aβ 16-23-Fragment geeignet ist.

4. Medikament nach Anspruch 1, wobei man bei dem Behandeln oder Durchführen einer Prophylaxe der Krankheit in Kombination mit dem Medikament einen Antikörper verabreicht, der an einem Epitop mit Aβ1-11 spezifisch an Aβ bindet.

5. Medikament nach Anspruch 4, wobei der Antikörper, der an einem Epitop innerhalb von Aβ 1-11 spezifisch an Aβ bindet, für die Verabreichung vor dem Aβ 16-23-Fragment geeignet ist.

6. Medikament nach einem der vorhergehenden Ansprüche, wobei die Krankheit durch Beeinträchtigung der kognitiven Fähigkeiten gekennzeichnet ist.

7. Medikament nach einem der vorhergehenden Ansprüche, wobei die Krankheit aus der Gruppe bestehend aus Morbus Alzheimer, Down-Syndrom und milder Beeinträchtigung der kognitiven Fähigkeiten ausgewählt ist.

8. Medikament nach einem der vorhergehenden Ansprüche, wobei man bei dem Behandeln oder Durchführen einer Prophylaxe der Krankheit die induzierten Antikörper in den Patienten verfolgt.

9. Medikament nach einem der vorhergehenden Ansprüche, wobei der Patient symptomfrei ist.

10. Medikament nach einem der vorhergehenden Ansprüche, wobei der Patient Symptome aufweist und das Medikament die Verschlechterung der Symptome des Patienten hemmt.

11. Medikament nach einem der vorhergehenden Ansprüche, wobei der Patient ein Lebensalter von unter 50 Jahren aufweist.

12. Medikament nach einem der vorhergehenden Ansprüche, wobei der Patient einen vererbten Risikofaktor, der Anfälligkeit für Morbus Alzheimer anzeigt, aufweist.

13. Medikament nach Anspruch 9, wobei der Patient fünf Jahre lang nach der ersten Durchführung der Behandlung oder Prophylaxe keine nachweisbaren Symptome entwickelt.

14. Medikament nach einem der Ansprüche 1-11 und 13, wobei der Patient keine bekannten Risikofaktoren für Morbus Alzheimer aufweist.

15. Medikament nach einem der vorhergehenden Ansprüche, das sich für die Verabreichung in einer Dosierung von mindestens 50 Mikrogramm des Fragments an einer Mehrzahl von Tagen eignet.

16. Medikament nach einem der vorhergehenden Ansprüche, das sich für die Verabreichung in mehrfachen Dosierungen über einen Zeitraum von mindestens drei Monaten eignet.

17. Medikament nach Anspruch 16, wobei die Dosierungen mindestens 50 Mikrogramm betragen.

18. Medikament nach einem der vorhergehenden Ansprüche, das weiterhin ein Adjuvans umfasst, das den von dem Fragment induzierten Antikörperspiegel erhöht.

19. Medikament nach einem der vorhergehenden Ansprüche, das sich für die intraperitoneale, orale, intranasale, subkutane, intramuskuläre, topische oder intravenöse Verabreichung eignet.

20. Medikament nach einem der vorhergehenden Ansprüche, wobei man bei dem Behandeln oder Durchführen einer Prophylaxe der Krankheit weiterhin den Patienten auf den Spiegel an induzierten Antikörpern im Blut des Patienten überwacht.

21. Medikament nach Anspruch 1, wobei es sich bei dem Träger um ein heterologes Polypeptid handelt.

22. Medikament nach Anspruch 1, wobei multiple Kopien des Fragments mit einem Trägermolekül unter Bildung eines Konjugats verknüpft sind.

23. Medikament nach Anspruch 1, wobei multiple Kopien des Fragments mit multiplen Kopien des Trägermoleküls, die miteinander verknüpft sind, verknüpft sind.

24. Medikament nach Anspruch 21, wobei das heterologe Polypeptid QYIKANSKFIGITEL (SEQ ID NO: 8) umfasst.

25. Medikament nach Anspruch 21, wobei das heterologe Polypeptid die Aminosäuresequenz AKXVAAWTLKAAA (SEQ ID NO: 11) umfasst.

26. Medikament nach Anspruch 21, wobei das Polypeptid eine T-Zellreaktion gegen das heterologe Polypeptid und dadurch eine B-Zellreaktion gegen das Fragment induziert.

27. Medikament nach Anspruch 1, wobei bei dem Behandeln oder Durchführen einer Prophylaxe weiterhin ein Adjuvans verabreicht wird, das den Titer und/oder die Bindungsaffinität der induzierten Antikörper relativ zu dem Titer und/oder der Bindungsaffinität der von dem Fragment induzierten Antikörper allein verstärkt.

28. Medikament nach Anspruch 27, das das Adjuvans und das Fragment umfasst.

29. Medikament nach Anspruch 27, wobei sich das Adjuvans für die Verabreichung vor dem Fragment eignet.

30. Medikament nach Anspruch 27, wobei sich das Adjuvans für die Verabreichung nach dem Fragment eignet.

31. Medikament nach Anspruch 27, wobei das Adjuvans aus der Gruppe bestehend aus Aluminiumsulfat, MPL, QS-21 und inkomplettem Freund'schen Adjuvans ausgewählt ist.

32. Medikament nach einem der vorhergehenden Ansprüche, das das Fragment in einer Dosierung von mehr als 10 Mikrogramm umfasst.

## Revendications

1. Médicament comprenant un fragment d'Aβ constitué d'Aβ16-23 (KLVFFAED) dans la forme humaine naturelle d'acides aminés ; dans lequel le fragment est lié à une molécule de porteur pour former un conjugué qui aide à obtenir une réponse immunitaire dirigée contre le fragment ;
pour effectuer le traitement ou la prophylaxie d'une maladie associée à des dépôts amyloïdes d'Aβ dans le cerveau d'un patient, les anticorps induits se liant spécifiquement à Aβ soluble chez le patient ce qui inhibe de cette manière la formation de dépôts amyloïdes d'Aβ dans le cerveau à partir de l'Aβ soluble et ce qui effectue de cette manière le traitement ou la prophylaxie de la maladie.

2. Médicament selon la revendication 1, traiter la maladie ou effectuer la prophylaxie de la maladie comprenant l'administration en association avec ledit médicament d'un fragment d'Aβ qui induit des anticorps se liant spécifiquement à Aβ au niveau d'un ou plusieurs épitopes au sein d'Aβ1-11.

3. Médicament selon la revendication 2, dans lequel le fragment d'Aβ qui induit des anticorps se liant spécifiquement à Aβ au niveau d'un épitope au sein d'Aβ1-11 est approprié pour une administration avant le fragment Aβ16-23.

4. Médicament selon la revendication 1, traiter la maladie ou effectuer la prophylaxie de la maladie comprenant l'administration en association avec ledit médicament d'un anticorps qui se lie spécifiquement à Aβ au niveau d'un épitope avec Aβ1-11.

5. Médicament selon la revendication 4, dans lequel l'anticorps qui se lie spécifiquement à Aβ au niveau d'un épitope au sein d'Aβ1-11 est approprié pour une administration avant le fragment Aβ16-23.

6. Médicament selon l'une quelconque des revendications précédentes, la maladie étant **caractérisée par** un trouble cognitif.

7. Médicament selon l'une quelconque des revendications précédentes, la maladie étant choisie dans le groupe constitué par la maladie d'Alzheimer, la trisomie 21 et un trouble cognitif léger.

8. Médicament selon l'une quelconque des revendications précédentes, traiter la maladie ou effectuer la prophylaxie de la maladie comprenant le suivi des anticorps induits chez le patient.

9. Médicament selon l'une quelconque des revendications précédentes, le patient étant asymptomatique.

10. Médicament selon l'une quelconque des revendications précédentes, le patient étant symptomatique et le médicament inhibant l'aggravation des symptômes du patient.

11. Médicament selon l'une quelconque des revendications précédentes, le patient étant âgé de moins de 50 ans.

12. Médicament selon l'une quelconque des revendications précédentes, le patient ayant un facteur de risque héréditaire indiquant une prédisposition à la maladie d'Alzheimer.

13. Médicament selon la revendication 9, le patient ne développant pas de symptômes détectables pendant cinq ans après que le traitement ou la prophylaxie est effectué pour la première fois.

14. Médicament selon l'une quelconque des revendications 1-11 et 13, le patient n'ayant pas de facteurs de risque connus pour la maladie d'Alzheimer.

15. Médicament selon l'une quelconque des revendications précédentes, qui est approprié pour une administration à une dose d'au moins 50 microgrammes du fragment sur plusieurs jours.

16. Médicament selon l'une quelconque des revendications précédentes, qui est approprié pour une administration en de multiples doses sur une durée d'au moins trois mois.

17. Médicament selon la revendication 16, les doses étant d'au moins 50 microgrammes.

18. Médicament selon l'une quelconque des revendications précédentes, qui comprend en outre un adjuvant qui augmente le taux d'anticorps induits par le fragment.

19. Médicament selon l'une quelconque des revendications précédentes, qui est approprié pour une administration intrapéritonéale, orale, intranasale, sous-cutanée, intramusculaire, topique ou intraveineuse.

20. Médicament selon l'une quelconque des revendications précédentes, traiter la maladie ou effectuer la prophylaxie de la maladie comprenant en outre le suivi du patient en ce qui concerne le taux d'anticorps induits dans le sang du patient.

21. Médicament selon la revendication 1, dans lequel le porteur est un polypeptide hétérologue.

22. Médicament selon la revendication 1, dans lequel de multiples copies du fragment sont liées à une molécule de porteur pour former un conjugué.

23. Médicament selon la revendication 1, dans lequel de multiples copies du fragment sont liées à de multiples copies de la molécule de porteur, qui sont liées les unes aux autres.

24. Médicament selon la revendication 21, dans lequel le polypeptide hétérologue comprend QYIKANSKFIGITEL (SEQ ID n° : 8).

25. Médicament selon la revendication 21, dans lequel le polypeptide hétérologue comprend la séquence d'acides aminés AKXVAAWTLKAAA (SEQ ID n° : 11).

26. Médicament selon la revendication 21, dans lequel le polypeptide induit une réponse des lymphocytes T dirigée contre le polypeptide hétérologue et de cette manière une réponse des lymphocytes B dirigée contre le fragment.

27. Médicament selon la revendication 1, traiter ou effectuer la prophylaxie comprenant en outre l'administration d'un adjuvant qui accroît le titre et/ou l'affinité de liaison des anticorps induits par rapport au titre et/ou à l'affinité de liaison des anticorps induits par le fragment seul.

28. Médicament selon la revendication 27, qui comprend l'adjuvant et le fragment.

29. Médicament selon la revendication 27, dans lequel l'adjuvant est approprié pour une administration avant le fragment.

30. Médicament selon la revendication 27, dans lequel l'adjuvant est approprié pour une administration après le fragment.

31. Médicament selon la revendication 27 dans lequel l'adjuvant est choisi dans le groupe constitué par l'alun, le MPL, la QS-21 et l'adjuvant incomplet de Freund.

32. Médicament selon l'une quelconque des revendications précédentes, qui comprend le fragment à une dose supérieure à 10 microgrammes.
